# EUROPEAN PATENT APPLICATION

(11) **EP 1 939 284 A1**
(43) Date of publication of application: **02.07.2008**
(21) Application number: 06798146.4
(22) Date of filing: 20.09.2006
(51) Int. Cl.: C12N 15/00, C07K 2/00, C12N 5/06, C12N 5/10, C12N 15/09, C12P 21/00

(54) **HOST CELLS FOR PRODUCTION OF RECOMBINANT PROTEIN**

(30) Priority: 20.09.2005 JP 2005272058
(71) Applicant: Taisho Pharmaceutical Co. Ltd., Tokyo 170-8633 (JP)
(72) Inventor: TAIRA, Hideharu, Morioka-shi, Iwate 0200133 (JP); YAMASHITA, Tetsuro, Morioka-shi Iwate; 0200114 (JP); MIYAZAKI, Masao, Asaka-shi Saitama; 3510014 (JP)
(74) Representative: Polypatent
(86) International application number: PCT/JP2006/318587
(87) International publication number: WO 2007/034809

(57) **Abstract**

It is an object of the present invention to provide host cells, which are able to proliferate in a serum free medium, which are able to synthesize and secrete a large amount of protein of interest, and which are able to produce a glycoprotein having a sugar chain structure. The present invention provides an established cell line, which is established from distal tubular epithelial cells derived from a mammal.

## Description

### TECHNICAL FIELD

The present invention relates to host cells used for production of a recombinant protein, and a use of the aforementioned host cells. The present invention particularly relates to host cells used for production of a recombinant protein established from a mammal, and a use of the aforementioned host cells.

### BACKGROUND ART

In the functional analysis of a gene whose functions are unknown, it is essential to obtain a recombinant protein encoded by the aforementioned gene. The currently used expression systems for production of recombinant proteins include a prokaryotic system, a eukaryotic system, and a cell-free system. Such prokaryotic systems used as hosts include: *Escherichia coli* and related bacteria (e.g. *E. coli,* bacteria of genus Pseudomonas, bacteria of genus Zymomonas, bacteria of genus Thermus, etc.); and *Bacillus subtilis* and related bacteria) (e.g. Actinomycetes, lactic acid bacteria, amino acid-producing bacteria, acetic acid bacteria, thermophilic bacteria, etc.). Such eukaryotic systems used as hosts include yeast, filamentous bacteria, cultured plant cells, cultured insect cells (baculovirus vector), and cultured animal cells.

Among the aforementioned expression systems, mass production systems of recombinant proteins using microorganisms such as *Escherichia coli* or yeast have been established, and several proteins have been used as medicaments. However, in eukaryotes, and in particular, in the case of human proteins, it has been known that addition of characteristic sugar chains and a complicated processing conducted after the synthesis of such human proteins are essential for activity of the proteins. Thus, there may be cases where it is difficult to produce functional proteins from microorganisms.

Moreover, even in a case where a protein does not undergo posttranslational modification, when a human gene is expressed in an *Escherichia coli* expression system, inclusion bodies may be often formed due to differences in codon usage or conformation of proteins. Thus, even if a gene can be obtained, an enormous time is required for obtaining the gene product (protein). This causes a bottleneck in many cases.

On the other hand, when a protein of interest is produced using insect cells or cultured animal cells, this is problematic in terms of (1) a low amount of production, (2) a limited range of genes to be expressed, (3) high costs, and the like. Accordingly, time and effort are required for obtaining a protein to be subjected to screening.

Furthermore, when a large-scale production of proteins is carried out using animal cells, this method has been problematic in the following respects. (1) In the case of transient expression of a protein, the protein of interest can be obtained in a short time. However, the amount of a protein that can be produced by a single transfection is limited, and a scale-up is not easy. Thus, large quantities of cells and transfection reagents are required. (2) Cells that highly express proteins over a long period of time can be obtained by producing a cell line where the introduced gene is stably expressed. However, this method has been problematic in that it takes a long time (several months) to produce a cell line into which a foreign gene has been stably introduced.

A glycoprotein with a molecular weight of 60 kDa named as "Cauxin" is egested in the urine of cats at a high concentration of approximately 1 mg/ml. It has been reported that Cauxin is generated in distal tubular epithelial cells and is then secreted from an apical membrane (Non-Patent Document 1).

Non-Patent Document 1: Miyazaki, M., Kamiie, K., Soeta, S., Taira, H., and Yamashita, T.: Molecular cloning and characterization of a novel carboxylesterase-like protein that is physiologically present at high concentration in the urine of domestic cats (Felis catus) Biochem. J., 370,101-110 (2003).

### DISCLOSURE OF THE INVENTION

### Problems to be Solved by the Invention

It is an object of the present invention to provide host cells, which are able to proliferate in a serum free medium, which are able to synthesize and secrete a large amount of protein of interest, and which are able to produce a glycoprotein having a sugar chain structure.

### Means for Solving the Problems

As a result of intensive studies directed towards achieving the aforementioned object, the present inventors have found that an established cell line, which is established from cat-derived distal tubular epithelial cells, is able to proliferate in a serum free medium, is able to synthesize and secrete a large amount of protein of interest, and is also able to produce a glycoprotein having a sugar chain structure. The present invention has been completed based on such findings.

Thus, the present invention provides an established cell line, which is established from distal tubular epithelial cells derived from a mammal.

Preferably, the established cell line of the present invention is used as a host cell for production of a recombinant protein.

Preferably, the established cell line of the present invention is derived from a cat.

Preferably, the established cell line of the present invention has a gene encoding a foreign immortality-imparting factor.

Preferably, the established cell line of the present invention has a foreign selective marker gene.

Preferably, the established cell line of the present invention is able to proliferate in a serum free medium.

Preferably, the established cell line of the present invention is a cell having an accession No. FERM BP-10643.

According to another aspect, the present invention provides a transformant, which is obtained by introducing a gene encoding a protein of interest to be expressed into the established cell line of the present invention as mentioned above.

According to further another aspect, the present invention provides a method for producing a recombinant protein using the established cell line of the present invention as mentioned above.

Preferably, a foreign gene is introduced into the established cell line of the present invention as mentioned above to produce transformed cells, the obtained transformed cells is cultured so as to allow the cells to produce a recombinant protein encoded by the foreign gene, and the produced recombinant protein can be recovered.

Preferably, the produced recombinant protein is modified with a sugar chain.

According to further another aspect, the present invention provides a recombinant protein, which is produced by the method of the present invention as mentioned above.

### BEST MODE FOR CARRYING OUT THE INVENTION

The embodiments of the present invention will be described in detail below.

The cells of the present invention are established cells which are established from mammal-derived distal (renal) tubular epithelial cells. Examples of such a mammal include cat, dog, rabbit, guinea pig, hamster, rat, mouse, and human. Of these, cat is preferable.

The cells of the present invention can be collected from cat kidney, for example. The kidney excised from a cat is fragmented in a Joklik buffer (111 mM NaCI, 24 mM NaHCO₃, 10 mM Na₂HPO₄, 5.4 mM KCl, 1 mM Mg₂SO₄, and 11 mM glucose), and the fragmented tissue section is treated with a collagenase digestive fluid. Thereafter, cell masses are removed from the thus fragmented cells. The cells are then recovered by centrifugation. Subsequently, the cells are suspended in Eagle's MEM medium 1 (Nissui), and distal tubular cells are then separated by Percoll density gradient centrifugation, so that the aforementioned cells can be recovered. The cells are washed with Eagle's MEM medium 1 so as to remove Percoll, and the resultant cells are then suspended in a DME-F12 medium, followed by culture on a collagen-coated dish.

An immortality-imparting factor and a selective marker gene are introduced into the thus isolated distal tubule-derived cells, so as to establish a cell line. The term "immortality-imparting factor" is used to mean a factor that is introduced into cells to immortalize them. Examples of such an immortality-imparting factor used herein include an Epstein-Barr virus (EBV) gene, a Simian virus 40 (SV40) T antigen gene, an SV40 large T antigen gene, an adenovirus E1A gene, an adenovirus E1B gene, a human papillomavirus (HPV) E6 gene, an HPV E7 gene, and a telomerase reverse transcriptase protein (hTERT) gene. As a selective marker gene, a drug resistance gene, an auxotrophic gene, and the like can be used. Examples of such genes used herein include a neomycin resistance gene, a puromycin resistance gene, a hygromycin resistance gene, and a zeocin resistance gene. A gene encoding an immortality-imparting factor and a selective marker gene can be introduced by a common transfection method. After completion of the transfection, the used medium is exchanged with a selective medium (in the case of introducing a drug resistance gene, a medium containing the aforementioned drug is used, for example), and a cell line, into which a selective marker gene has been stably introduced and which is able to stably proliferate, is selected, thereby establishing the cell line of the present invention.

As an example of the cells of the present invention, FKD cells (identification name: FKD) obtained in the example as given below were deposited with the National Institute of Advanced Industrial Science and Technology, an Independent Administrative Institution under the Ministry of Economy, Trade and Industry (at the AIST Tsukuba Central 6, Higashi 1-1-1, Tsukuba, Ibaraki, Japan, (Post code: 305-8566)) under accession No. FERM P-20605 on July 27,2005. Thereafter, this deposition was transferred to an international deposition under "Budapest Treaty on the International Recognition of the Deposit of Microorganisms for the Purposes of Patent Procedure" on July 19, 2006 under an accession No. FERM BP-10643.

The thus established cell line of the present invention can be cultured by a common method for culturing animal cells. As a medium used in the culture, commonly used RPM11640 medium [The Journal of the American Medical Association, 199, 519 (1967)], Eagle's MEM medium [Science, 122, 501(1952)], DMEM medium [Virology, 8, 396 (1959)], DMEM-F12 medium, and the like can be used. Otherwise, a medium obtained by adding fetal bovine serum or the like to such a medium can also be used. However, since the cells of the present invention are able to proliferate even in a serum free medium, it is not necessary to add serum. It may also be possible to add insulin, transferrin, serine, or the like to such a medium, as appropriate.

In general, culturing can be carried out under conditions such as pH 6 to 8, a temperature between 30°C and 40°C, and the presence of 5% CO₂. In addition, during such culturing, antibiotics such as neomycin may be added to a medium, as necessary. Culturing may also be carried out on a collagen-coated dish.

In order to allow a recombinant protein of interest to express in the cells of the present invention, first, a gene encoding such a recombinant protein is inserted into a site downstream of a promoter in a suitable expression vector. Thereafter, the expression vector, into which the aforementioned gene has been inserted, is introduced into the cells of the present invention.

As an expression vector, a vector, which is capable of autonomously replicating in the cells of the present invention or is capable of being incorporated into the chromosome and which comprises a promoter at a site for transcribing the aforementioned gene of interest, can be used.

Examples of such an expression vector include pCH110 (PL), pcDNAI, pcDM8 (Funakoshi Corp.), pcDNAI/AmP (Invitrogen), pREP4 (Invitrogen), pCDNA3 (Invitrogen), pCMVscript (Stratagene), pSG5 (Stratagene), pZeoSV2 (Invitrogen), pEF1/V5 HisA/B/C (Invitrogen), pEF-Bos-Myc, pREP4 (Invitrogen), and pBK-RSV (Stratagene).

Any type of promoter can be used, as long as it can be expressed in animal cells. Examples of such a promoter include a cytomegalovirus (human CMV) IE (immediate early) gene promoter, an SV40 early promoter, a retrovirus promoter, a metallothionein promoter, a heat shock promoter, and an SRα promoter. Moreover, a human CMV IE gene enhancer may also be used together with such a promoter.

As a method for introducing a recombinant expression vector into the cells of the present invention, a common method for introducing DNA into animal cells can be used. Examples of such a method include an electroporation method, a calcium phosphate method, and a lipofection method.

In addition, as a method for expressing a recombinant protein, direct expression as well as secretory production, expression of a fusion protein and the like can be carried out.

When a recombinant protein is allowed to express in the cells of the present invention, a protein, to which sugar or a sugar chain is added, can be obtained.

A transformant obtained by introducing a gene encoding a protein of interest to be expressed into the cells of the present invention is cultured in a preferred medium, and a protein of interest is produced and accumulated in the culture. Thereafter, the protein is collected from the culture, so as to produce the protein of interest. The thus produced protein is also included in the scope of the present invention.

As a method for culturing the transformant of the present invention in a medium, a common method used in the culture of a host can be applied.

As a medium for culturing a transformant, commonly used RPM11640 medium [The Journal of the American Medical Association, 199, 519 (1967)], Eagle's MEM medium [Science, 122, 501 (1952)], DMEM medium [Virology, 8, 396 (1959)], DMEM-F12 medium, and the like can be used. Otherwise, a medium obtained by adding fetal bovine serum or the like to such a medium can also be used. However, since the cells of the present invention are able to proliferate even in a serum free medium, it is not necessary to add serum.

In general, culturing can be carried out for 1 to 7 days under conditions such as pH 6 to 8, a temperature between 30°C and 40°C, and the presence of 5% CO₂. In addition, during such culturing, antibiotics such as neomycin may be added to a medium, as necessary.

In order to isolate and purify a recombinant protein of interest from the culture of the transformant of the present invention, a common method for isolating and purifying a protein may be used.

When a recombinant protein is expressed in cells in a soluble state, for example, the cells are recovered by centrifugation after completion of the culturing, and they are then suspended in a water-based buffer. Thereafter, the cells are crushed using an ultrasonic disintegrator, a French press, a Manton Gaulin homogenizer, a Dyno mill, etc., so as to obtain a cell-free extract. The cell-free extract is centrifuged to obtain a supernatant. Thereafter, the obtained supernatant is subjected to common methods for isolating and purifying a protein; namely, a solvent extraction method, a salting-out method using ammonium sulfate, a desalination method, a precipitation method using an organic solvent, anion exchange chromatography using a resin such as diethylaminoethyl (DEAE) sepharose or DIAION HPA-75 (manufactured by Mitsubishi Kasei Corp.), cation exchange chromatography using a resin such as S-Sepharose FF (manufactured by Pharmacia), hydrophobic chromatography using a resin such as butyl sepharose or phenyl sepharose, a gel filtration method using a molecular sieve, affinity chromatography, a chromatofocusing method, electrophoresis such as isoelectric electrophoresis, etc. Such methods are used singly or in combination, so as to obtain a purified sample.

When a recombinant protein is expressed in cells in the form of an insoluble form, the cells are recovered and are then crushed, followed by centrifugation, so as to obtain a precipitated fraction. Thereafter, a protein is recovered from the precipitated fraction by an ordinary method, and the insoluble form of the protein is solubilized with a protein denaturant. The solubilized solution is diluted or dialyzed to be a solution that contains no protein denaturants or a solution that contains a protein denaturant whose concentration is too low to denature the protein, so that the protein is allowed to have a normal three-dimensional structure. Thereafter, the same isolation and purification methods as described above are applied, so as to obtain a purified sample.

When a recombinant protein or a derivative thereof such as a sugar-modified derivative thereof is secreted outside the cells, the protein or a derivative thereof such as a sugar chain-added derivative thereof can be recovered in the culture supernatant. That is to say, the culture is treated by the aforementioned methods such as centrifugation to obtain a soluble fraction. Thereafter, a purified sample can be obtained from the soluble fraction by the same above isolation and purification methods.

The present invention will be described more in detail in the following examples. However, these examples are not intended to limit the scope of the present invention.

### EXAMPLES

### Example 1: Isolation of cat renal tubular epithelial cells

Cat distal tubule-derived epithelial cells were isolated as follows according to the method described in Soshiki Baiyo no Gijyutsu (2nd edit.) Nihon Soshiki Baiyo Gakkai Hen, Asakura Shoten, 7-4 Jinsaibo Baiyo Ho (pp. 141-145) (Tissue Culture Techniques (2nd edit.) edited by the Japanese Tissue Culture Association, Asakura Shoten, 7-4 Renal Cell Culture Method (pp. 141-145)). A cat was subjected to anesthesia, and the kidney was aseptically excised. The excised kidney was fragmented with a knife in a Joklik buffer (111 mM NaCl, 24 mM NaHCO₃, 10 mM Na₂HPO₄, 5.4 mM KCl, 1 mM Mg₂SO₄, and 11 mM glucose). 30 ml of a 0.02% collagenase digestive fluid was added to 1 g of the fragmented tissue section in a 100-ml Erlenmeyer flask, and the obtained mixture was then stirred with a stirrer for 30 minutes. Thereafter, a supernatant was discarded, and a fresh digestive fluid was added, followed by a further reaction for 30 minutes. The fragmented cells were filtrated through gauze to remove cell masses, and the cells were then recovered by centrifugation at 1,000 rpm for 5 minutes. The cells were suspended in Eagle's MEM medium 1 (Nissui), and distal tubular epithelial cells were separated and recovered by Percoll density gradient centrifugation. The cells were washed with Eagle's MEM medium 1 so as to remove Percoll, and the resultant cells were then suspended in a DME-F12 medium, followed by culturing on a collagen-coated dish.

### Example 2: Establishment of FKD cells

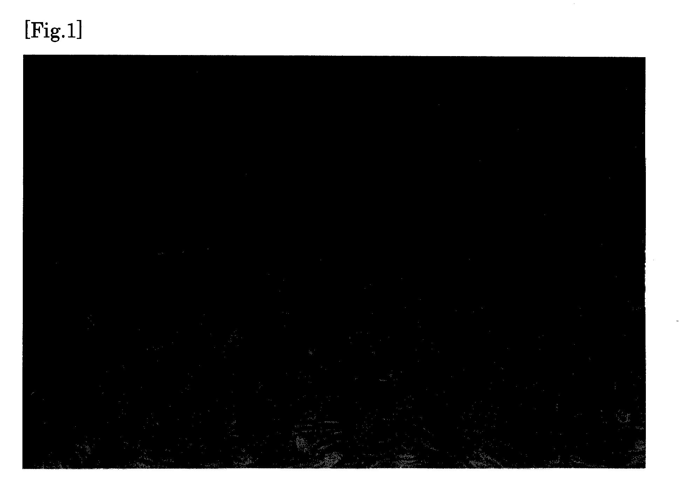

Using Lipofectamine-Plus (Invitrogen), pSV3-neo (ATCC37150) was transfected to the distal tubule-derived cells isolated in Example 1. Forty-eight hours after the transfection, the medium was exchanged with a G418-containing medium, and a stable mutation introduced cell line was then selected. The obtained FKD cells are shown in Figure 1. The proliferated cells were subjected to a passage, and the resultant cells were then preserved in liquid nitrogen.

### Example 3: Subculture (passage) of FKD cells

FKD cells were subcultured in a DMEM-F12 (+supplement) medium on a collagen-coated dish at 37°C in the presence of 5% CO₂. When the cells became 80% to 90% confluent, the medium was removed. The cells were washed with PBS/EDTA, and 1 ml of PBS/EDTA was then added thereto, followed by incubation at 37°C in the presence of 5% CO₂ for 5 minutes. Subsequently, the cells were washed with 0.1% trypsin-PBS/EDTA, followed by incubation under the same above conditions. Thereafter, the resultant cells were suspended in a DMEM-F12 (+supplement) medium, and the suspension was diluted and then cultured in a fresh collagen-coated dish. As a supplement (GIBCO ITS-X supplement), a product comprising insulin, transferrin and serine was used.

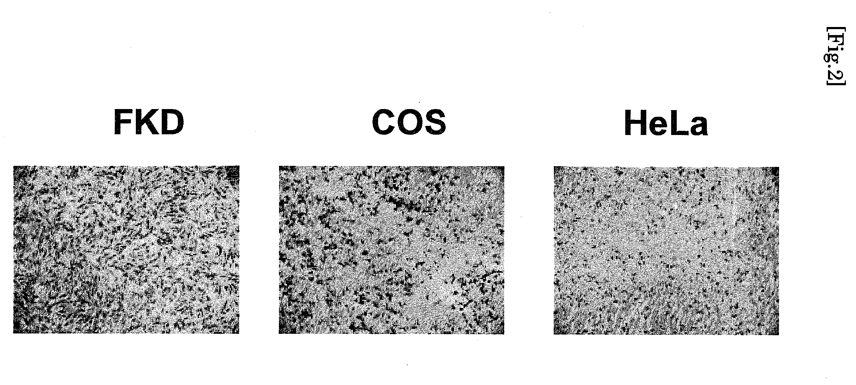

### Example 4: Transfection experiment (gene transduction efficiency; Figure 2)

In order to study transfection efficiency, an expression vector pCH110 (Pharmacia) produced by binding LacZ to an SV40 promoter was used. Transfection was carried out using Lipofectamine-Plus (Invitrogen). As cells, FKD cells, COS cells, and HeLa cells were used. Twenty-four hours after the transfection, the medium was removed, and the cells were then rinsed with 1.5 ml of PBS once. Thereafter, 1.5 ml of a fixative solution was added thereto, and the mixture was then left at rest for 10 minutes, so as to fix the cells. Thereafter, the cells were rinsed with 1.5 m1 of PBS twice, and 1.25 ml of a staining solution (β-gal Staining Kit (Invitrogen)) was then added thereto, followed by incubation. Three hours later, expression efficiency was confirmed from the ratio of the stained cells. The results are shown in Figure 2. The FKD cells of the present invention exhibited a gene transduction efficiency higher than those of COS cells and HeLa cells.

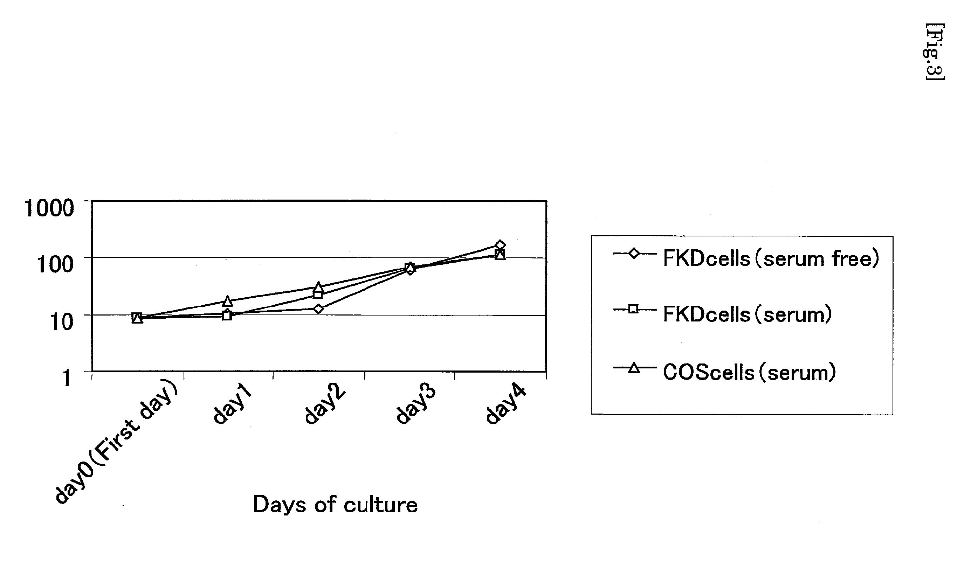

### Example 5: Production of growth curves of FKD cells and COS cells (Figure 3)

The growth of FKD cells in a serum free medium and in a serum medium and the growth of COS cells in a serum medium were analyzed. Confluent FKD cells (6-cm collagen-coated dish (4.0 × 10⁶ cell)) were dispersed on the same above dish, resulting in a cell number of 1 : 10 (4.0 × 10⁵ cells). Likewise, the same cells were dispersed in a serum medium on a 3.5-cm dish, resulting in a cell number of 1 : 10 (1.6×10⁵ cells). The cells were treated with PBS/EDTA and 0.1% trypsin-PBS/EDTA, and they were then suspended in 2 ml of a serum free medium (the cells in a serum medium were suspended in 1 ml of a serum medium). The cells were recovered and were then centrifuged at 700 rpm for 10 minutes. Thereafter, a supernatant was discarded, and PBS and a trypan blue solution were added to the residue. The mixture was fully stirred. The suspension was placed on a hemacytometer, and the number of cells per dish (surviving cells and all cells) was counted twice. At that time, PBS and a trypan blue solution were added, so that the number of cells contained in a compartment (1 mm x 1 mm) to be observed could become approximately 100. The number of cells on two dishes was counted per day, and the mean value was obtained. Such measurement was carried out every 24 hours for 4 days. The amounts of PBS and trypan blue suspended were changed to facilitate observation of the cells every day. The depth of a compartment was 0.1 mm. Thus, the number of cells in 1 ml was obtained by multiplying the volume of such a compartment (0.1 mm3) by 10⁴, and the value obtained by multiplying the aforementioned number of cells by the amount of a PBS-trypan blue solution/1000 (ml) was defined as the number of actually suspended cells. After completion of the observation, the obtained values were plotted on a semilogarithmic graph, so as to produce a growth curve. Likewise, COS cells were dispersed on a 3.5-cm dish, resulting in a cell number of 1 : 10 (1.6 x 10⁵ cells), and they were then observed for 4 days. From the logarithmic growth phase in the growth curve of each type of cells, a doubling time was obtained.

The results are shown in Figure 3. As a result, the doubling times of FKD in a serum free medium, FKD in a serum medium, and COS in a serum medium were 19.0,20.7, and 21.8 hours, respectively.

### Example 6: Transfection of mouse interferon α and purification of expressed protein

FKD cells were cultured on a dish with a diameter of 10 cm (collagen-coated dish, IWAKI) up to a subconfluent state (approximately 90% or more). The medium was removed, and 12 ml of DMEM-F12 (+ Supplement; penicillin/streptomycin were excluded) was then added to the cells, followed by incubation. At the same time, 12 µg of pHGGS-Mu-IFNα1 acting also as an expression plasmid of mouse interferon (hereinafter abbreviated as IFN), and a mixture obtained by adding 1.5 ml of OPTI-MEM to the pHGGS-Mu-IFNα1, were produced. In addition, a mixture obtained by mixing 36 µl of Lipofectamine 2000 (Invitrogen) and 1.5 ml of OPTI-MEM was also produced. After leaving at rest for 5 minutes, a Lipofectamine 2000 solution was added to the DNA solution, and the mixture was then left at rest for 20 minutes. Twenty minutes later, the reaction solution was added to the incubated cells, and the obtained mixture was then stirred. Eight hours later, the medium was exchanged with 10 ml of DMEM-F12 (+ Supplement), and it was then incubated for 16 hours.

Twenty-four hours after the transfection, the operation to recover the medium was repeated, and 200 ml of the medium was recovered as a sample of IFN purification. The recovered medium was passed through a CPG column (column size: 10 ml) obtained by filling a 10-ml syringe with CPG (Controlled-Pore Glass), so that the medium was adsorbed on the column. At that time, attention was paid that the flow rate of the medium poured into the column was kept at 6 to 10 ml. After 200 ml of the medium was passed through the column, the inside of the column was washed with 100 ml of 1 x PBS. Thereafter, in order to elute IFN, 25 ml of 0.4 M glycine-HCl (pH 3.5) was first poured therein, and 25 ml of 0.4 M glycine-HCl (pH 2.0) was then poured therein. Mouse IFNα is eluted with a solution of pH 2.0, and IFNβ is eluted with a solution of pH 3.5. In the present experiment, since IFNα was synthesized in FKD cells, IFNα was eluted to an eluted fraction of pH 2.0. In addition, since IFN has the property of adsorbing on a glass, a plastic container was used as a container for such an eluted fraction.

After completion of the elution, 25 ml of each eluted fraction was dialyzed with 1 x PBS. At that time, 1 x PBS was exchanged twice. After completion of the dialysis, the resultant fraction was concentrated by AQUACIDE II (CALBIOCHEM), and it was then dialyzed with 1 x PBS again. Thereafter, the fraction was centrifuged by Ultra-4 centrifugal filter unit (Amicon) at 5,000 rpm for 30 minutes, and thus each eluted fraction was finally concentrated to 100 µl.

5 µl of a 5 x SDS-PAGE sampling buffer (reductive condition) was added to 20 µl of each eluted fraction, and the obtained mixture was then boiled for 5 minutes to create a sample. Thereafter, SDS-PAGE was carried out. At that time, 25 µl of the sample was supplied to each lane of 15% gel, and the total amount of sample was supplied at 15 mA. The electrophoresed gel, six filters previously impregnated with a blotting buffer, and a PVDF filter were laminated in the order of the three filters, the PVDF filter, the gel, and the three filters from the bottom. Thereafter, transfer was carried out at a constant current of 1.5 mA/cm² for 1 hour 30 minutes. After completion of the transfer, the resultant was immersed in a blocking buffer, so that a blocking treatment was carried out at 4°C overnight. After completion of the blocking treatment, an anti-mouse IFNα polyclonal antibody (rabbit) was diluted 500 times, and it was used as a primary antibody. The filter was reacted with the antibody in a plastic pack at room temperature for 1 hour. Thereafter, the filter was washed with TBS-T (5 minutes x 5). Protein A-HRP (2000 times diluted) was used as a secondary antibody. The filter was reacted with the secondary antibody at room temperature for 1 hour. After the filter was washed with TBS-T (5 minutes x 5), the filter was reacted with ECL plus (Amersham Biosciences), and was then exposed to an X-ray film. As a result of the analysis by Western blot, a band (approximately 20 kDa) was detected at a position of IFNα to which a sugar chain was added. Thus, it was found that sugar chain-added IFNα was secreted.

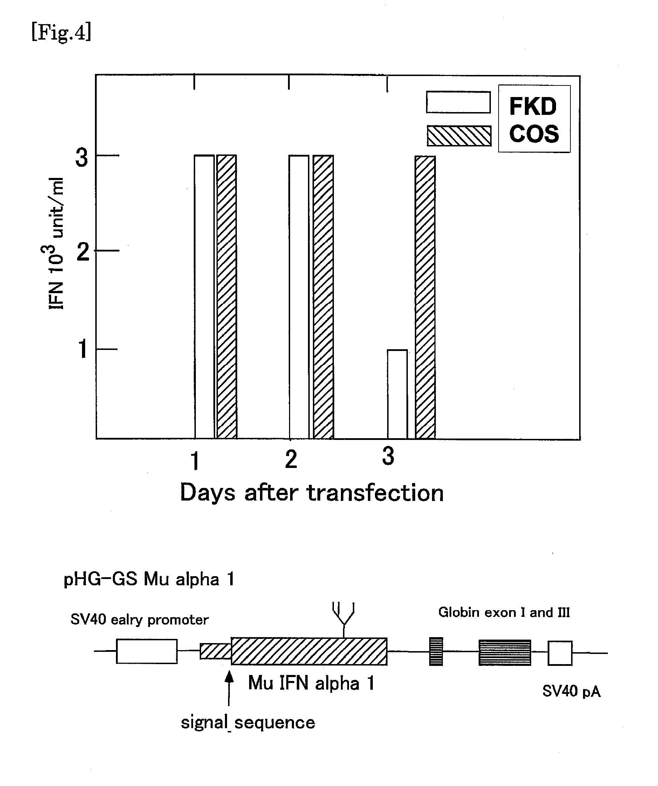

### Example 7: Measurement of biological activity of IFN (CPE (cytopathic effect) Assay; Figure 4)

An IFN expression vector was transfected to FKD cells, and the thus transfected medium was added to L cells (Flow laboratories) so as to cause viral infection of the cells. After that, if the cells did not cause CPE due to viral infection, it can be said that active IFN existed in the medium, namely, activated IFN was secreted outside the FKD cells.

In order to conduct a CPE assay, diluted VSV was added to L cells, and twenty-four hours later, the cells were observed. As a result, it was confirmed that CPE sufficiently occurs by 100 to 200 times dilution. An IFN expression vector was transfected to FKD cells. The medium was exchanged with a fresh one for 5 days, and the cells were then recovered. Thereafter, the cells were centrifuged at 3,000 rpm for 5 minutes, and a supernatant was then recovered and used as a sample.

L cells were dispersed on a 96-well plate, resulting in a ratio of 1 : 2. After the cells had proliferated, 50 µl of IFN was added to the uppermost well in the longitudinal lane, and it was then stirred 10 times. Thereafter, 50 µl of the reaction solution was transferred to a lower well. This operation was continued until the solution reached the lowermost well, so as to produce a lane in which IFN was diluted 3 times in a stepwise manner. The same above operation was carried out also on a sample obtained after completion of the aforementioned transfection, so that individual lanes were produced.

Six hours later, the medium was removed from each well, and 100 µl of VSV that was 200 times diluted with 0.5% NCS-MEM was added to each well. At the same time, a control well of VSV was also prepared. After completion of the culturing for 24 hours, if it was confirmed that L cells in the VSV control well had caused CPE, the cells were stained with crystal violet dye and were then dried. Thereafter, the ratio of surviving cells stained into violet was observed. A spot wherein 50% of total cells had been degenerated was identified from the wells of each lane, and the approximate amount of IFN released (unit/ml) was assumed. In the case of COS cells, since it had been confirmed that IFN is released to outside the cells, the same operation was carried out also on such COS cells. A comparison was made between the two types of cells. The results are shown in Figure 4. The 50% CPE spot of FKD cells was almost the same as that of COS cells. Thus, it was considered that the secretion quantities of FKD cells were equivalent to those of COS cells (10,000 U/ml).

### Example 8: Transfection of Cauxin and expression of protein

Using pcDNA6.2/GW/D-TOPO (Invitrogen), a Cauxin expression vector was produced. By using the cDNA of Cauxin as a template (it is to be noted that the nucleotide sequence of Cauxin gene has been registered at the NCBI under Accession No. AB045377.), a full-length open reading frame was amplified by PCR (KOD-Plus from TOYOBO was used in PCR) using the following primers:
F-Primer : CACCATGAGTGGGATGTGGGTGCA (SEQ ID NO: 1); and
RV Primer : TCAGGGGACAATGGTATTCA (SEQ ID NO: 2), and
the amplified product was then incorporated as an insert into the 950 n position of the vector.

For transfection, the lipofection method was applied. FKD cells were cultured on a 3-cm dish up to a subconfluent state (approximately 80% to 90%), and the medium was then removed. 1.5 ml of 5% FBS-DMEM-F12 (-penicillin/streptomycin) was added, followed by incubation. At the same time, 2 µl of CMV-cauxin (1 µg/µl) and a mixture obtained by adding 250 µl of OPTI-MEM to the CMV-cauxin were produced. In addition, a mixture obtained by mixing 6 µl of Lipofectamine 2000 (Invitrogen) and 250 µl of OPTI-MEM was also produced. After leaving at rest for 5 minutes, a Lipofectamine 2000 solution was added to the DNA solution, and the mixture was then left at rest for 20 minutes. Twenty minutes later, the reaction solution was added to the incubated cells, and the obtained mixture was then stirred. Twenty hours later, the medium was exchanged with DMEM-F12 and 5% FBS-DMEM-F12, and it was then incubated for 8 hours. Eight hours later, the medium (1.5 ml) was recovered, and 1 ml of a soluble buffer was added to each dish. Thereafter, a sonic treatment was carried out for 5 minutes, and the reaction product was then centrifuged at 15,000 rpm for 10 minutes. Thereafter, a supernatant was recovered, and it was used as a cell lysate (900 µl). With regard to the medium, the medium was treated at 3,000 rpm for 5 minutes, so as to produce a medium, from which dead cells had been eliminated.

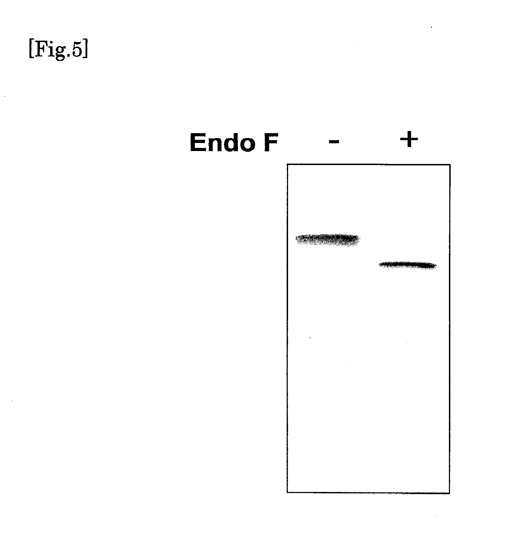

### Example 9: Treatment with glycopeptidase F (GPF) (Figure 5)

After immunoprecipitation with a cauxin C-terminal peptide antibody, 20 µl ofMQ water was added to a sample to be subjected to a glycosidase treatment, and the obtained mixture was then stirred. Thereafter, 10 µl of the mixed solution was transferred into another tube. 10 µl of a denature buffer (+2-ME) was added thereto, and the mixture was then boiled at 100°C for 3 minutes. Thereafter, 20 µl of a stabilized solution and 52 µl of MQ water were added thereto, and the mixture was stirred. Thereafter, 8 µl (0.5 mU/µl) of GPF (TAKARA) was added to the mixture, and the obtained mixture was then stirred, followed by incubation at 37°C for 20 hours. Thereafter, 25 µl of 5 x SDS-PAGE sampling buffer (reductive condition) was added, and the obtained mixture was then boiled for 5 minutes to create a sample. Thereafter, a supernatant was recovered, and SDS-PAGE was carried out. At that time, 10 µl of the sample was supplied to each lane of 10% gel, and the total amount of sample was supplied at 15 mA. After completion of the electrophoresis, three filters, a membrane, a gel, and three filters were laminated in this order. Transfer was carried out at a constant current of 1.5 mA/cm² for 1 hour 30 minutes. After completion of the transfer, the resultant was immersed in a blocking buffer, so that a blocking treatment was carried out at 4°C overnight.

After completion of the blocking treatment, a cauxin C-terminal peptide antibody (8,000 times diluted) was used as a primary antibody. The filter was reacted with the antibody in a plastic pack at room temperature for 1 hour. Thereafter, the filter was washed with TBS-T (5 minutes x 5). Protein A-HRP (8,000 times diluted) was used as a secondary antibody. The same treatment as in the case of the primary antibody was carried out. After the filter was washed with TBS-T (5 minutes x 5), the filter was was reacted with ECL (purchased from Amersham Biosciences) and was exposed to an X-ray film. The results are shown in Figure 5. From the results as shown in Figure 5, it was confirmed that a sugar chain was added to a cauxin protein allowed to express in the FKD cells of the present invention.

### Example 10: Suspension culturing of FKD cells

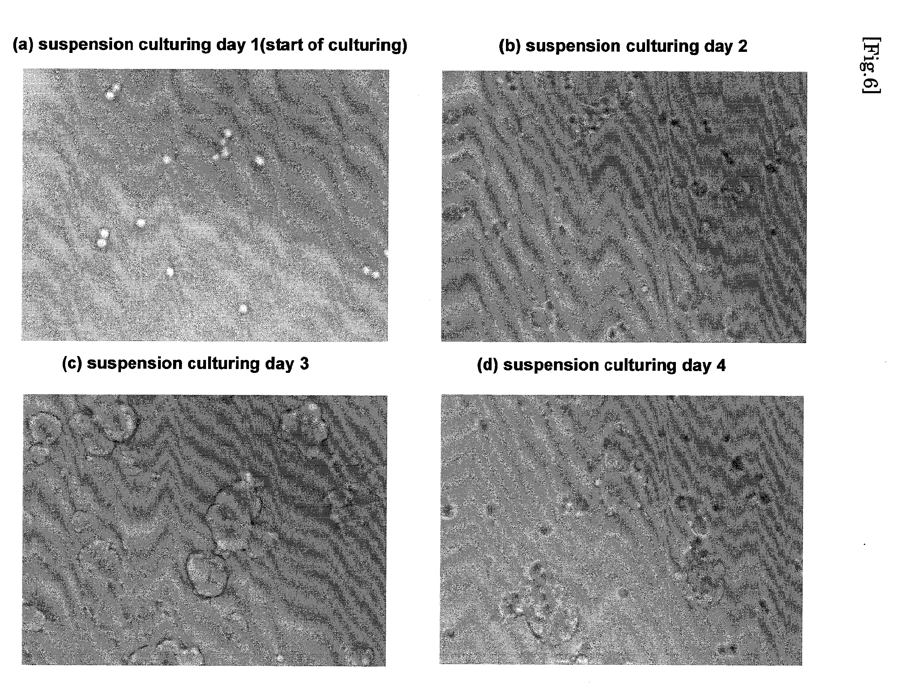

FKD cells were cultured on four 10-cm dishes up to an almost confluent state (medium: DMEM F-12 (10565-018, GIBCO; 5% FCS). The medium was removed, and the cells were washed with 5 ml of PBS-EDTA. Thereafter, 1 ml of 0.1 % trypsin/PBS-EDTA was added thereto, and the mixture was then incubated in a CO₂ incubator for 4 minutes. The cells released from the dishes were recovered using a P1000 PIPETMAN, and they were then placed in a 50-ml centrifuge tube that contained 10 ml of a serum free medium (OPTI PRO SFM, 12309-019, GIBCO) comprising 5 mg of a soybean trypsin inhibitor (Wako 202-09221). The cells were then centrifuged at 100 g for 4 minutes. Thereafter, a supernatant was removed, and the cells were suspended in 10 ml of a serum free medium (as described above), and the suspension was then centrifuged at 100 g for 4 minutes. Thereafter, a supernatant was removed, and the cells were then resuspended in 10 ml of a serum free medium. The suspension was added to a spinner flask (S-flask 4500-250, TAITEC) that contained 90 ml of a serum free medium comprising 1 ml of Pluronic F-68 (11905-031, GIBCO), and the cells were then cultured in a CO₂ incubator (5% CO₂, 37°C) at a stirrer rotation number of 90 rpm. The state of the cells from day 1 to day 4 after initiation of the suspension culturing is shown in Figure 6.

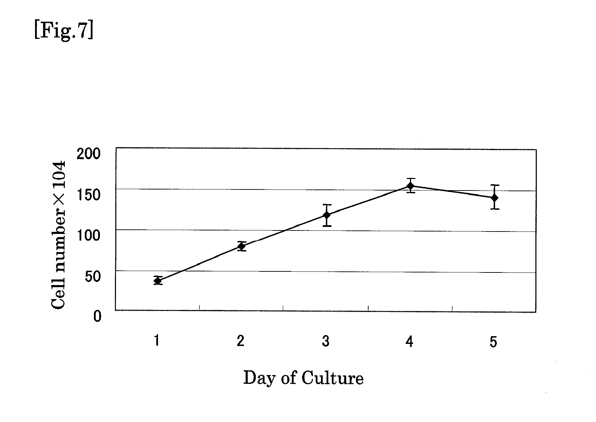

The number of cells was measured as follows. 1 ml of a culture solution was centrifuged at 100 g x 4 minutes, so as to recover cells. The recovered cells were washed with 1 ml of PBS-EDTA, and 1 ml of 0.1% trypsin/PBS-EDTA was added thereto. Thereafter, the obtained mixture was left at room temperature for 5 minutes, so that cell masses were dispersed. Thereafter, the number of cells was measured using a hemacytometer. The results obtained by measuring the number of cells from day 1 to day 5 after initiation of the suspension culturing are shown in Figure 7. As shown in Figure 7, cell growth was observed until day 4 to day 5 after initiation of the suspension culturing.

### INDUSTRIAL APPLICABILITY

The established cell line of the present invention is able to proliferate in a serum free medium, is able to synthesize and secrete a large amount of protein of interest, and is able to produce a glycoprotein having a sugar chain structure. Accordingly, the established cell line of the present invention is useful as a host cell used for production of a recombinant protein. In particular, using the established cell line of the present invention as a host cell, it becomes possible to produce a large amount of protein of interest at a low cost in the form of the protein having biological activity.

### BRIEF DESCRIPTION OF THE DRAWINGS

Figure 1 shows FKD cells.
Figure 2 shows the results obtained by analyzing the gene transduction efficiency of FKD cells, COS cells, and HeLa cells. To each type of cells, lacZ was introduced, and the cells were then stained with X-gal. Cell density was the same in all of the three types of cells.
Figure 3 shows the results obtained by comparing the growth of FKD cells with the growth of COS cells. The rhombus indicates FKD cells (serum free), the square indicates FKD cells (serum), and the triangle indicates COS cells (serum).
Figure 4 shows the results obtained by analyzing the expression of Mu IFN-α1 in FKD cells and in COS cells.
Figure 5 shows the results obtained by analyzing the expression of glycoprotein Cauxin. Using an anti-Cauxin antibody, Western blotting was carried out regarding a case where Endo F (sugar chain cleavage enzyme) was added to a FKD cell extract, in which Cauxin gene had been expressed, and a case where no Endo F was added to such a FKD cell extract. Endo F (Endoglycosidase F) specifically cleaves the binding site (GlcNAc-Asn bond) of an N-glycoside sugar chain with a protein. This enzyme cleaves such a binding site regardless of the structure of a sugar chain portion, except for a sugar chain in which fucose binds to GluNAc at a reducing terminal via α1-3 bond.
Figure 6 shows the state of FKD cells on day 1 (a), day 2 (b), day 3 (c), and day 4 (d) after initiation of the suspension culturing of the cells.
Figure 7 shows the results obtained by measuring the number of FKD cells from day 1 to day 5 after initiation of the suspension culturing of the cells.

P22188EP.ST25.txt
## Claims

1. An established cell line, which is established from distal tubular epithelial cells derived from a mammal.

2. The established cell line according to claim 1, which is used as a host cell for production of a recombinant protein.

3. The established cell line according to claim 1 or 2, which is derived from a cat.

4. The established cell line according to any one of claims 1 to 3, which has a gene encoding a foreign immortality-imparting factor.

5. The established cell line according to any one of claims 1 to 4, which has a foreign selective marker gene.

6. The established cell line according to any one of claims 1 to 5, which is able to proliferate in a serum free medium.

7. The established cell line according to any one of claims 1 to 6, which is a cell having an accession No. FERM BP-10643.

8. A transformant, which is obtained by introducing a gene encoding a protein of interest to be expressed into the established cell line of any of claims 1 to 7.

9. A method for producing a recombinant protein using the established cell line of any of claims 1 to 7.

10. The method according to claim 9, which comprises: introducing a foreign gene into the established cell line of any of claims 1 to 7 to produce transformed cells; culturing the obtained transformed cells, so as to allow the cells to produce a recombinant protein encoded by the foreign gene; and recovering the produced recombinant protein.

11. The method according to claim 9 or 10, wherein the produced recombinant protein is modified with a sugar chain.

12. A recombinant protein, which is produced by the method of any of claims 9 to 11.


SEQUENCE LISTING

<110>  Taisho Pharmaceutical Co., Ltd.

<120>  HOST CELLS USED FOR PRODUCTION OF RECOMBINANT PROTEIN

<130>  P22188EP

<140>  EP 06798146.4
<141>  2006-09-20

<150>  JP 272058/2005
<151>  2005-09-20

<160>  2

<170>  PatentIn version 3.3

<210>  1
<211>  24
<212>  DNA
<213>  Artificial

<220>
<223>  Synthetic DNA

<400>  1
caccatgagt gggatgtggg tgca                                              24


<210>  2
<211>  20
<212>  DNA
<213>  Artificial

<220>
<223>  Synthetic DNA

<400>  2
tcaggggaca atggtattca                                                   20